# EUROPÄISCHE PATENTSCHRIFT

(11) **EP 0 968 687 B1**
(45) Veröffentlichungstag und Bekanntmachung des Hinweises auf die Patenterteilung: **10.05.2006**
(21) Anmeldenummer: 99111516.3
(22) Anmeldetag: 14.06.1999
(51) Int. Cl.: A61C 19/04, A61C 13/00, G01B 11/24

(54) **3D-Kamera zur Erfassung von Oberflächenstrukturen, insbesondere für zahnmedizinische Zwecke**
3D-camera for measurement of surface structures, in particular for dentistry
Camera tridimensionelle pour mesurer des structures superficielles, en particulier pour l'art dentaire

(30) Priorität: 30.06.1998 DE 19829278
(43) Veröffentlichungstag der Anmeldung: 05.01.2000
(73) Patentinhaber: Sirona Dental Systems GmbH, 64625 Bensheim (DE)
(72) Erfinder: Pfeiffer, Joachim Dr.rer.nat. Dipl.Phys., 64625 Bensheim (DE); Schwotzer, Axel Dipl.Phys., 64521 Gross-Gerau (DE)
(74) Vertreter: Sommer, Peter

(56) Entgegenhaltungen:
- DE-A1- 3 810 455
- DE-A1- 4 218 219
- DE-U1- 9 013 454

## Beschreibung

Die Erfindung betrifft eine 3D-Kamera zur Erfassung von Oberflächenstrukturen eines Aufnahmeobjekts mittels Triangulation, insbesondere für zahnmedizinische Zwecke. Diese 3D-Kamera umfaßt Mittel zum Erzeugen eines Lichtstrahlenbündels, welches über einen Projektionsstrahlengang aus einer ersten Richtung zu einem Aufnahmeobjekt lenkbar ist und das Aufnahmeobjekt ausleuchtet, und einen Beobachtungsstrahlengang mit einem Bildsensor zum Empfang des vom Aufnahmeobjekt rückgestreuten Lichts. Der Projektions- und Beobachtungsstrahlengang nehmen einen Winkel zueinander ein, wodurch ein dreidimensionales Vermessen möglich ist. Weiterhin sind in dem Projektionsstrahlengang Mittel zum Erzeugen eines auf das Aufnahmeobjekt projizierten Referenzmusters angeordnet.

Eine derartige 3D-Kamera (d.h. eine Kamera zur Erfassung dreidimensionaler Strukturen) ist in der Zeitschrift "Technisches Messen: Sensoren, Geräte, Systeme", Ausgabe Juni 1996, Seiten 254 bis 261, Oldenbourg-Verlag B3020, offenbart. Der technische Inhalt wird vollständig in diese Anmeldung miteinbezogen.

Aus der US-Patentschrift 4,575,805 ist eine 3D-Kamera bekannt, mit der eine Oberflächenstruktur eines Aufnahmeobjektes in Hinsicht auf Höhen- bzw. Tiefenunterschiede erfaßt werden kann. Diese bekannte 3D-Kamera besitzt einen Projektions- und einen Beobachtungsstrahlengang, die einen Winkel zu einer optischen Achse der 3D-Kamera einnehmen. Im Projektionsstrahlengang ist eine Lichtquelle zum Aussenden eines Lichtstrahlenbündels in Richtung zu einem Aufnahmeobjekt angeordnet. Das vom Aufnahmeobjekt reflektierte Licht wird durch den Beobachtungsstrahlengang zu einem Bildsensor der 3D-Kamera gelenkt. Die Signale des Bildsensors können einer Auswerteeinheit zugeführt werden, so daß ein Bild von der Oberflächenstruktur auf einer Anzeigevorrichtung erstellt werden kann. Diese 3D-Kamera eignet sich insbesondere zur Erfassung einer Kavität eines Zahnes.

Auch aus der EP-A-0 250 993 ist eine solche 3D-Kamera bekannt. Zur Bestimmung der Höhen- bzw. Tiefenunterschiede der Oberflächenstruktur sind Mittel zur Erzeugung eines Referenzmusters vorgesehen, derart, daß das Referenzmuster auf die Oberflächenstruktur projizierbar ist. Anhand des von der Oberflächenstruktur reflektierten Lichtes, das auf den Bildsensor auftrifft, und in Verbindung mit einer Auswerteelektronik zur Ausführung eines in den oben genannten Dokumenten näher erläuterten, mit "Phase-shifting-Triangulation" bezeichneten Verfahrens, kann die Oberflächenstruktur in Hinsicht auf Höhen- bzw. Tiefenunterschiede berechnet und auf einem Monitor als pseudodreidimensionales Bild dargestellt werden.

Aus den oben genannten Dokumenten ist offenbart, daß vorzugsweise ein linienförmiges Referenzmuster auf die Oberflächenstruktur projiziert wird, welches von einer LCD-Anordnung oder einem mechanischen Gitter erzeugbar ist. Bei einer vorgegebenen Periode des Referenzmusters ergibt sich der Eindeutigkeitsbereich, d.h. der Bereich, in dem ein Höhenunterschied zweier Objektpunkte eindeutig erfaßt werden kann, nach folgender Formel:

Eindeutigkeitsbereich = Periodendauer des Referenzmusters/Tangens des Winkels, den der Projektionsstrahlengang und der Beobachtungsstrahlengang zueinander einnehmen.

Limitiert durch elektrisches Rauschen und andere Effekte ist die erzielbare Meßgenauigkeit immer ein bestimmter Bruchteil des Eindeutigkeitsbereiches (typ. 1/100). Folglich ist bei einer großen Periodendauer der Eindeutigkeitsbereich groß, wobei jedoch der Höhenunterschied zweier Objektpunkte nicht so genau erfaßt werden kann. Bei einer kleinen Periodendauer ist der Eindeutigkeitsbereich klein, jedoch kann der Höhenunterschied zweier Objektpunkte mit großer Genauigkeit erfaßt werden.

Da es wünschenswert ist, auch größere Höhenunterschiede zweier Objektpunkte eindeutig und genau erfassen zu können, wurde in der DE 90 13 454 U1 eine 3D-Kamera vorgeschlagen, bei welcher im Projektionsstrahlengang Mittel zum Erzeugen eines ersten Referenzmusters und eines zweiten Referenzmusters auf das Aufnahmeobjekt vorhanden sind. Dadurch, daß Referenzmuster mit vorzugsweise unterschiedlicher Periode auf das Aufnahmeobjekt projiziert werden, kann gegenüber der Verwendung nur eines Referenzmusters ein wesentlich größerer Höhenunterschied zweier Objektpunkte eindeutig erfaßt werden.

Nachteilig ist hier, daß entweder eine Überlagerung des ersten Gitters über das zweite erforderlich ist, wodurch eine schlechtere Meßgenauigkeit insgesamt erzielt wird, oder daß eine lange Aufnahmedauer erforderlich ist. Insgesamt ist der konstruktive Aufwand sehr hoch.

Um weiterhin auch bei ungünstigen Oberflächenstrukturen eine Vermessung der Oberflächenstruktur an dieser Stelle zu ermöglichen, werden weitere Mittel zum Erzeugen eines weiteren Lichtstrahlenbündels vorgeschlagen, welches aus einer zweiten, zur ersten unterschiedlichen Richtung über einen weiteren Projektionsstrahlengang zu dem Aufnahmeobjekt lenkbar ist. Dadurch kann die Oberflächenstruktur aus verschiedenen Richtungen ausgeleuchtet werden, wobei vorgeschlagen wird, daß in jedem Projektionsstrahlengang ein Mittel zum Erzeugen eines Referenzmusters angeordnet wird.

Nachteilig ist hier, daß der apparative Aufwand gerade für handbetätigte 3D-Kameras hoch ist und somit ein handliches Gerät nur schwer zu realisieren ist.

Diesen Nachteil weist auch die WO 98/11 403 A1 auf, aus welcher ein Verfahren und eine Vorrichtung zur dreidimensionalen Vermessung von Objekten durch optische Aufnahmen, aufprojizierte Muster und Triangulationsberechnungen bekannt ist, bei dem die Projektionseinheit für das Muster und die Aufnahmeeinheit voneinander getrennt aufgebaut sind und im Verlauf des Vermessungsvorgangs unabhängig voneinander positioniert bzw. geführt werden können.

Die Aufgabe der Erfindung besteht folglich darin, die Meßeindeutigkeit bei großen Höhenunterschieden zu erreichen, ohne dabei die Meßgenauigkeit zu verringern, und gleichwohl den apparativen Aufwand gering zu halten.

Die Aufgabe wird gelöst durch eine 3D-Kamera zur Erfassung von Oberflächenstrukturen eines Aufnahmeobjekts mittels Triangulation, insbesondere für zahnmedizinische Zwecke, mit
- Mitteln zum Erzeugen eines Lichtstrahlenbündels, um über einen Projektionsstrahlengang das Aufnahmeobjekt anzuleuchten,
- einem Bildsensor, um über einen Beobachtungsstrahlengang rückgestreutes Licht vom Aufnahmeobjekt zu empfangen, sowie
- mit Mitteln im Projektionsstrahlengang zum Erzeugen eines auf das Aufnahmeobjekt projizierten Musters.

Die 3D-Kamera enthält erfindungsgemäß Mittel im Projektionsstrahlengang und/oder im Beobachtungsstrahlengang zur Veränderung des Triangulationswinkels, der durch den Winkel zwischen dem Schwerpunktstrahl des Projektionsstrahlengangs und dem Schwerpunktstrahl des Beobachtungsstrahlengangs definiert ist.

Die Mittel zur Veränderung des Triangulationswinkels bewirken eine Veränderung des Schwerpunktstrahls des Projektions- und/oder Beobachtungsstrahlenganges. Durch die Vorsehung dieser Mittel läßt sich unter Beibehaltung einer kompakten Bauweise die Meßeindeutigkeit bei großen Höhenunterschieden herstellen.

Gemäß einer Weiterbildung ist das Mittel zur Veränderung des Triangulationswinkels eine in Form und/oder Lage veränderbare Blende. Durch Öffnen oder Schließen der Blende verschiebt sich der Schwerpunktstrahl, wobei hierzu eine asymmetrische Blende erforderlich ist.

Gemäß einer anderen Weiterbildung ist das Mittel zur Veränderung des Triangulationswinkels ein Abschattungsplättchen, welcher in den Projektions- und/oder Beobachtungsstrahlengang eingebracht wird, insbesondere in Form eines durch einen Hubmagneten betriebenen Fähnchens.

In einer weiteren Ausgestaltung kommt eine Blende zum Einsatz, die in ihrer Lichtdurchlässigkeit veränderliche Flüssigkeitskristalle aufweist.

Weiterhin ist es vorteilhaft, sowohl den Verlauf des Schwerpunktstrahls des Projektionsstrahlenganges als auch den Verlauf des Schwerpunktstrahls des Beobachtungsstrahlenganges durch geeignete Mittel zu verändern, um die Abweichung des Triangulationswinkels stärker zu beeinflussen.

Als Triangulationswinkel eignet sich insbesondere ein Winkel von 3° bis 15°, wobei der Triangulationswinkel um 3 bis 50% veränderbar ist.

Eine weitere Lösung der gestellten Aufgabe besteht in einem Verfahren zur Erfassung von Oberflächenstrukturen eines Aufnahmeobjekts mittels Triangulation, insbesondere für zahnmedizinische Zwecke. Bei dem erfindungsgemäßen Verfahren werden in enger zeitlicher Reihenfolge mindestens zwei 3D-Vermessungen desselben Objektes durchgeführt, wobei zwischen den beiden Vermessungen der Triangulationswinkel zwischen dem Schwerpunktstrahl des Projektionsstrahlengangs und dem Schwerpunktstrahl des Beobachtungsstrahlengangs geringfügig geändert wird. Diese Änderung des Triangulationswinkels führt zu unterschiedlichen Aufnahmen des zu vermessenden Objekts, aus denen die dreidimensionale Gestalt des Objekts berechnet werden kann.

Vorteilhafterweise wird der Triangulationswinkel um einen Faktor zwischen 0,7 und einem Wert kleiner als 1 oder um einen Faktor zwischen einem Wert größer als 1 und 1,3 verändert. Das bedeutet, daß der Triangulationswinkel bis höchstens zum 0,7-fachen seines Ausgangswertes verkleinert oder bis höchstens zum 1,3-fachen seines Ausgangswertes vergrößert wird. In diesem Bereich läßt sich eine ausreichende Erhöhung des Eindeutigkeitsbereichs erzielen, wobei eine ausreichende Qualität der Signale Voraussetzung ist. Die Qualität der Signale muß um so besser sein, je näher der Faktor der Änderung des Triangulationswinkels bei 1 liegt.

Vorteilhafterweise erfolgt die Änderung des Triangulationswinkels nach der ersten Vermessung durch eine die Lage des Schwerpunktstrahls verschiebende Abschattung oder Öffnung des Projektions- und/oder Beobachtungsstrahlengangs mittels einer in Form und/oder Lage veränderbaren Blende.

In der Zeichnung sind die wesentlichen Merkmale der Erfindung dargestellt. Es zeigt die
- Fig. 1: einen prinzipiellen Strahlengang einer 3D-Kamera und die
- Fig. 2: einen gemessenen Höhenverlauf.

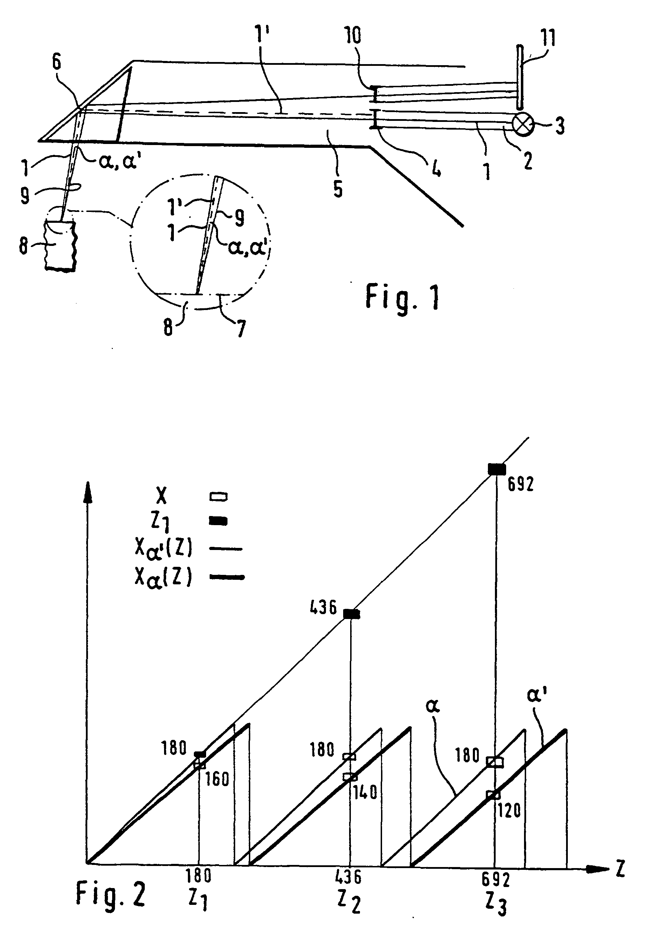

In Fig. 1 wird ein Projektionsstrahlengang 1 durch ein erstes Lichtstrahlenbündel 2 definiert, das von Mitteln 3 erzeugbar ist. Die Mittel 3 können beispielsweise eine LED und eine Optik aufweisen. Der Projektionsstrahlengang 1 ist durch den Schwerpunktstrahl dargestellt. Unter dem Schwerpunktstrahl versteht man den Strahl, der bezogen auf die Querschnittsfläche und Intensität des Lichtstrahlenbündels 2 den Mittelwert bildet. Genauer bedeutet dies, daß sich die Lage des Schwerpunktstrahls in einer Querschnittsfläche des Lichtbündels durch Mittelung der mit der jeweiligen Lichtintensität an einem Querschnittspunkt gewichteten Querschnittspunktkoordinaten ergibt. In einem Lichtstrahlenbündel homogener Intensität und von kreisförmiger Gestalt verläuft der Schwerpunktstrahl durch den Kreismittelpunkt.

Das Lichtstrahlenbündel 2 des Projektionsstrahlenganges 1 tritt durch eine Blende 4 in ein Prismenrohr 5, aus welchem das Lichtbündel nach Umlenkung mittels eines Prismas 6 in einem vorgegebenen Winkel zu der Längsachse des Prismenrohrs 5 austritt. Das über das Prisma 6 aus dem Prismenrohr 5 austretende Lichtstrahlenbündel, dargestellt durch den Schwerpunktstrahl, trifft auf die Oberfläche 7 eines zu vermessenden Objektes 8 und wird dort rückgestreut.

Das rückgestreute Licht durchläuft einen Beobachtungsstrahlengang 9. Der Schwerpunktstrahl des Beobachtungsstrahlengangs 9 schneidet die Oberfläche 7, wobei zwischen dem Projektionsstrahlengang und dem Beobachtungsstrahlengang ein als Triangulationswinkel bezeichneter Winkel α eingeschlossen ist. Das vom Aufnahmeobjekt 8 rückgestreute Licht wird über den Beobachtungsstrahlengang 9 wiederum über das Prisma 6 umgelenkt und über das Prismenrohr 5 und eine zweite Blende 10 einem Bildsensor 11 zugeführt. Der Bildsensor 11 wandelt die empfangenen Lichtsignale in elektrische Signale um, die einer aus den eingangs genannten Dokumenten bekannten Vorrichtung zur Verarbeitung der Signale zugeführt werden, so daß Daten erhalten werden, aus denen ein Bild der Oberflächenstruktur des Aufnahmeobjektes 8 erstellt werden kann.

Die zur Abbildung der Objekte auf den Bildsensor nötigen optischen Elemente sind aus Vereinfachungsgründen nicht dargestellt, ebenso die zur Abbildung des Gitters auf das Objekt notwendigen optischen Elemente und das Gitter selbst. Dieser Aufbau ist auch in der vorstehend erwähnten Zeitschrift "Technisches Messen", Seite 257, Bild 6, dargestellt und beschrieben.

Wesentlich für den Gegenstand der vorliegenden Anmeldung ist die Anordnung von Mitteln zur Verschiebung des Schwerpunktstrahls im Projektions- bzw.

Beobachtungsstrahlengang 1 bzw. 9. In der Zeichnung ist hierzu die Blende 4 so ausgeführt, daß sie in einem unteren Bereich abgeschattet werden kann, so daß sich der Schwerpunktstrahl 1 nach oben verschiebt, dargestellt durch die gestrichelte Linie 1'. Folgt man dem Projektionsstrahlengang 1' bei teilweise abgeschatteter Blende 4, so wird klar, daß sich der Triangulationswinkel α verringert hat, weshalb dieser Winkel als α' bezeichnet ist.

Die Abschattung der Blende bewirkt die Verschiebung des Schwerpunktstrahls, weshalb die von dem Bildsensor 11 empfangenen Lichtsignale gegenüber den bei geöffneter Blende gemessenen Lichtsignale abweichen. In welcher Art und Weise die Verschiebung des Schwerpunktstrahls erfolgt, ist für das grundsätzliche Prinzip der Erfindung zunächst ohne Bedeutung. Die Abschattung der Blende könnte aber beispielsweise durch einen mittels einen Hubmagneten angetriebenen Schieber erfolgen, der zur Erhöhung der Wiederholgenauigkeit gegen einen Anschlag bewegt wird. Alternativ sind in Form und/oder Lage veränderbare Blenden vorstellbar oder auch LCD-Feld-gesteuerte Blendenlöcher. Die optimale Ausnutzung des vorhandenen Platzes ist ein wesentlicher Faktor für die Wahl des zur Veränderung des Schwerpunktstrahles einzusetzenden Mittels.

Im Folgenden soll erläutert werden, wie sich aus zwei Aufnahmen mit geringfügig unterschiedlichen Triangulationswinkeln eindeutige Meßresultate errechnen lassen, auch über den ursprünglichen Eindeutigkeitsbereich der Einzelaufnahmen hinaus.

In Fig. 2 sind die gemessenen Höhenwerte xα, xα', wie sie bei den beiden erfindungsgemäßen Meßaufnahmen entstehen, über dem realen Höhenverlauf z des vermessenen Objekts in einem Diagramm aufgetragen.

Die Uneindeutigkeit der einzelnen Meßaufnahmen führt dabei zu der "Sägezahnform" des Diagramms. Die Periode des "Sägezahns" ist der Eindeutigkeitsbereich (E) der Einzelaufnahme. Man erkennt, daß bei großen Werten von z die Differenz der Meßwerte xα und xα' groß ist. Die Differenz erlaubt also einen Rückschluß darauf, in welchem Eindeutigkeitsbereich (Ordnung) der Objektpunkt liegt. Ist die Ordnung bekannt, kann der absolute Höhenwert z aus xα und xα' berechnet werden, wie für die Höhen Z1, Z2, Z3 in Fig. 2 gezeigt. Dazu wird nur das richtige Vielfache von E auf den Wert xα addiert. Durch eine solche Verrechnung der Werte xα und xα' aus den Einzelaufnahmen wird also die Uneindeutigkeit der Einzelaufnahmen beseitigt. Die Doppelaufnahme ist in einem vielfachen Bereich eindeutig, und zwar ist der sich ergebende Eindeutigkeitsbereich der Doppelaufnahme um den Faktor α/(α-α') größer als der der Einzelaufnahmen, wie sich durch Überlegung nachvollziehen läßt. Mit den typischen Werten, die dem erfindungsgemäßen Vorschlag zugrunde liegen, ergibt sich eine Erweiterung um den Faktor 10.

Die Meßgenauigkeit (Rauschen), wie sie durch die erste Messung unter Triangulationswinkel α gegeben ist, ändert sich dabei nicht, da auf den Meßwert nur die feste Größe E mehrfach addiert wird. Dies gilt selbst dann, wenn die Qualität der zweiten Aufnahme z.B. aufgrund geringer Intensität des Lichtes reduziert ist.

Bewegungen der Kamera relativ zum Objekt zwischen den beiden Meßaufnahmen beeinflussen die Meßgenauigkeit aus gleichem Grunde ebenfalls nicht, sie können höchstens dazu führen, daß ein falsches Vielfaches von E addiert wird.

Die Freiheit der Doppeltriangulationstechnik von Mehrdeutigkeiten erlaubt es auch, Absolutmessungen über die Lage des Meßobjektes relativ zur Kamera zu machen.

Diese können auch z.B. zur Feinkorrektur von Fehlern in der optischen Abbildung oder auch von Positionierfehlern des Anwenders genutzt werden.

## Patentansprüche

1. 3D-Kamera zur Erfassung von Oberflächenstrukturen eines Aufnahmeobjekts mittels Triangulation, insbesondere für zahnmedizinische Zwecke, mit
- Mitteln (3) zum Erzeugen eines Lichtstrahlenbündels, um über einen Projektionsstrahlengang (1, 1') das Aufnahmeobjekt (8) anzuleuchten,
- einem Bildsensor, um über einen Beobachtungsstrahlengang (9) rückgestreutes Licht vom Aufnahmeobjekt (8) zu empfangen, sowie
- mit Mitteln im Projektionsstrahlengang (1, 1') zum Erzeugen eines auf das Aufnahmeobjekt projizierten Musters,
**gekennzeichnet durch** Mittel (4, 10) im Projektionsstrahlengang (1, 1') und/oder dem Beobachtungsstrahlengang (9) zur Veränderung des Triangulationswinkels, der **durch** den Winkel zwischen dem Schwerpunktstrahl des Projektionsstrahlengangs (1, 1') und dem Schwerpunktstrahl des Beobachtungsstrahlengangs (9) definiert ist.

2. 3D-Kamera nach Anspruch 1, **dadurch gekennzeichnet, daß** das Mittel zur Veränderung des Triangulationswinkels eine in Form und/oder Lage veränderbare Blende (4, 10) ist, durch die der Verlauf des Schwerpunktstrahls veränderbar ist.

3. 3D-Kamera nach Anspruch 1 oder 2, **dadurch gekennzeichnet, daß** das Mittel zur Veränderung des Triangulationswinkels in einem Abschattungsplättchen besteht, welches in den Projektions- und/oder Beobachtungsstrahlengang (1, 1', 9) eingebracht wird, insbesondere in Form eines durch einen Hubmagneten betriebenen Fähnchens.

4. 3D-Kamera nach Anspruch 2, **dadurch gekennzeichnet, daß** die Blende in ihrer Lichtdurchlässigkeit veränderliche Flüssigkristalle aufweist.

5. 3D-Kamera nach einem der Ansprüche 1 bis 4, **dadurch gekennzeichnet, daß** sowohl der Verlauf des Schwerpunktstrahls des Projektionsstrahlengangs (1, 1') als auch der Verlauf des Schwerpunktstrahls des Beobachtungsstrahlengangs (9) durch geeignete Mittel zur Veränderung des Triangulationswinkels veränderbar ist.

6. 3-D-Kamera nach einem der Ansprüche 1 bis 5, **dadurch gekennzeichnet, daß** der Triangulationswinkel im Bereich von 3° bis 15° liegt.

7. 3-D-Kamera nach einem der Ansprüche 1 bis 6, **dadurch gekennzeichnet, daß** der Triangulationswinkel um 3 bis 50 % veränderbar ist.

8. Verfahren zur Erfassung von Oberflächenstrukturen eines Aufnahmeobjekts (8) mittels Triangulation, insbesondere für zahnmedizinische Zwecke, wobei auf das Aufnahmeobjekt (8) mittels eines Lichtstrahlenbündels ein Muster projiziert wird, das durch Mittel im Projektionsstrahlengang (1,1') in dem Lichtstrahlenbündel erzeugt wird, wobei weiterhin das vom Aufnahmeobjekt (8) zurückgestreute Licht über einen Beobachtungsstrahlengang (9) von einem Bildsensor empfangen wird, **dadurch gekennzeichnet, daß** in enger zeitlicher Reihenfolge mindestens zwei 3D-Vermessungen desselben Aufnahmeobjektes (8) durchgeführt werden, wobei zwischen den beiden Vermessungen mit Mitteln zur Veränderung des Triangulationswinkels der Triangulationswinkel (α,α') zwischen dem Schwerpunktstrahl des Projektionsstrahlengangs (1, 1') und dem Schwerpunktstrahl des Beobachtungsstrahlengangs (9) geringfügig geändert wird.

9. Verfahren nach Anspruch 8, **dadurch gekennzeichnet, daß** der Triangulationswinkel bis höchstens zum 0,7-fachen seines Ausgangswertes verkleinert oder bis höchstens zum 1,3-fachen seines Ausgangswertes vergrößert wird.

10. Verfahren nach einem der Ansprüche 8 oder 9, **dadurch gekennzeichnet, daß** die Änderung des Triangulationswinkels nach der ersten Vermessung durch eine die Lage des Schwerpunktstrahls verschiebende Abschattung oder Öffnung des Projektions- und/oder des Beobachtungsstrahlengangs mittels einer in Form und/oder Lage veränderbaren Blende (4, 10) erfolgt.

## Claims

1. A 3D camera for the acquisition of surface structures of an object to be optically imaged by means of triangulation, particularly for dental purposes, comprising
- means (3) for the generation of a bundle of light rays for the purpose of illuminating the object to be imaged (8) via an optical projection path (1, 1'),
- an image sensor adapted to receive, via an optical viewing path (9), back-scattered light from the object to be imaged (8), and
- means in the optical projection path (1, 1') for the creation of a pattern projected onto the object to be imaged,
**characterized by** means (4, 10) in the optical projection path (1, 1') and/or the optical viewing path (9) for altering the triangulation angle defined by the angle between the center-of-mass beam of the optical projection path (1, 1') and the center-of-mass beam of the optical viewing path (9).

2. A 3D camera as defined in claim 1, **characterized in that** said means for altering the triangulation angle is a diaphragm (4, 10) which is capable of changing its shape and/or position and by means of which the course of the center-of-mass beam can be altered.

3. A 3D camera as defined in claim 1 or claim 2, **characterized in that** the means for altering the triangulation angle consists of an obstruction platelet, which is introduced into the optical projection path and/or the optical viewing path trajectory (1, 1', 9), particularly in the form of a flaglet operated by a lifting magnet.

4. A 3D camera as defined in claim 2, **characterized in that** the diaphragm has variably translucent liquid crystals.

5. A 3D camera as defined in any one of claims 1 to 4, **characterized in that** both the course of the center-of-mass beam of the optical projection path (1, 1') and the course of the center-of-mass beam of the optical viewing path (9) can be modified by suitable means for altering the triangulation angle.

6. A 3D camera as defined in any one of claims 1 to 5, **characterized in that** the triangulation angle ranges from 3 ° to 15 °.

7. A 3D camera as defined in any one of claims 1 to 6, **characterized in that** the triangulation angle is variable to an extent of from 3 to 50 %.

8. A method for the acquisition of surface structures of an object (8) to be optically imaged by means of triangulation, particularly for dental purposes, wherein a pattern is projected onto the object to be imaged (8) by means of a bundle of light rays, which pattern is generated by means in the optical projection path (1, 1') in the bundle of light rays, and wherein the light backscattered from the object to be imaged (8) is received by an image sensor via an optical viewing path (9), **characterized in that** at least two 3D scans of the same object to be imaged (8) are carried out successively within a short period of time, and between said two scans slight modification of the triangulation angle (α, α') between the center-of-mass beam of the optical projection path (1, 1') and the center-of-mass beam of the optical viewing path (9) is effected with means for altering the triangulation angle.

9. A method as defined in claim 8, **characterized in that** the triangulation angle is reduced by not more than 0.7 times its starting value or is increased by not more than 1.3 times its starting value.

10. A method as defined in claim 8 or claim 9, **characterized in that** the alteration of the triangulation angle following the first scan is effected by a diaphragm (4, 10) which is variable in shape and/or position and which causes shadowing or opening of the optical projection path and/or the optical viewing path so as to displace the center-of-mass beam.

## Revendications

1. Caméra tridimensionnelle pour détecter des structures de surface d'un objet acquis au moyen de la triangulation, notamment dans des applications en médecine dentaire, comprenant
- des moyens (3) pour générer un faisceau de rayons lumineux pour éclairer l'objet acquis (8) par le biais d'un trajet de rayons de projection (1, 1'),
- un capteur d'image pour recevoir la lumière rétrodiffusée par l'objet acquis (8) par le biais d'un trajet de rayons d'observation (9), et
- comprenant des moyens dans le trajet de rayons de projection (1, 1') pour générer un modèle projeté sur l'objet acquis,
**caractérisée par** un moyen (4, 10) dans le trajet de rayons de projection (1, 1') et/ou le trajet de rayons d'observation (9) pour modifier l'angle de triangulation qui est défini par l'angle entre le rayon du barycentre du trajet de rayons de projection (1, 1') et le rayon du barycentre du trajet de rayons d'observation (9).

2. Caméra tridimensionnelle selon la revendication 1, **caractérisée en ce que** le moyen pour modifier l'angle de triangulation est un obturateur (4, 10) dont la forme et/ou la position est modifiable et par le biais duquel il est possible de modifier le tracé du rayon du barycentre.

3. Caméra tridimensionnelle selon la revendication 1 ou 2, **caractérisée en ce que** le moyen pour modifier l'angle de triangulation se compose d'une plaquette d'obscurcissement qui est introduite dans le trajet de rayons de projection et/ou d'observation (1, 1', 9), notamment sous la forme d'un fanion entraîné par un électroaimant.

4. Caméra tridimensionnelle selon la revendication 2, **caractérisée en ce que** l'obturateur présente des cristaux liquides dont la transparence est variable.

5. Caméra tridimensionnelle selon l'une des revendications 1 à 4, **caractérisée en ce que** le tracé du rayon du barycentre du trajet de rayons de projection (1, 1') ainsi que le tracé du rayon du barycentre du trajet de rayons d'observation (9) sont modifiables par des moyens appropriés pour modifier l'angle de triangulation.

6. Caméra tridimensionnelle selon l'une des revendications 1 à 5, **caractérisée en ce que** l'angle de triangulation est compris dans la plage entre 3° et 15°.

7. Caméra tridimensionnelle selon l'une des revendications 1 à 6, **caractérisée en ce que** l'angle de triangulation est modifiable de 3 à 50 %.

8. Procédé pour détecter des structures de surface d'un objet acquis (8) au moyen de la triangulation, notamment dans des applications en médecine dentaire, un modèle étant projeté sur l'objet acquis (8) au moyen d'un faisceau de rayons lumineux, lequel est généré par des moyens dans le trajet de rayons de projection (1, 1') dans le faisceau de rayons lumineux, la lumière rétrodiffusée par l'objet acquis (8) étant en outre reçue par un capteur d'image par le biais d'un trajet de rayons d'observation (9), **caractérisé en ce qu'**au moins deux mesures tridimensionnelles du même objet acquis (8) sont effectuées dans un ordre chronologique rapproché, l'angle de triangulation (α, α') entre le rayon du barycentre du trajet de rayons de projection (1, 1') et le rayon du barycentre du trajet de rayons d'observation (9) étant légèrement modifié entre les deux mesures avec des moyens de modification de l'angle de triangulation.

9. Procédé selon la revendication 8, **caractérisé en ce que** l'angle de triangulation est réduit jusqu'à un minimum de 0,7 fois sa valeur initiale et augmenté jusqu'à un maximum de 1,3 fois sa valeur initiale.

10. Procédé selon l'une des revendications 8 ou 9, **caractérisé en ce que** la modification de l'angle de triangulation s'effectue après la première mesure par un obscurcissement ou une ouverture du trajet de rayons de projection et/ou d'observation au moyen d'un obturateur (4, 10) dont la forme et/ou la position est modifiable qui déplace la position du rayon du barycentre.
